Europäisches Patentamt

**European Patent Office**

**Office européen des brevets**

(11) Numéro de publication: **0 093 062 B1**

(12) **FASCICULE DE BREVET EUROPEEN**

(45) Date de publication de fascicule du brevet: **11.03.92**

(21) Numéro de dépôt: **83400826.0**

(22) Date de dépôt: **26.04.83**

(51) Int. Cl.⁵: **C12N 15/00**, C12N 1/20, C12N 1/16, C12R 1/20, //A01N63/02,C12R1/07, C12N15/75

(54) **ADN contenant une séquence codant pour une protéine du cristal ou un polypeptide doué de propriétés insecticides, micro-organismes transformés par de tels ADN, compositions contenant lesdites protéines du cristal, polypeptide ou micro-organismes.**

(30) Priorité: **26.04.82 FR 8207201**

(43) Date de publication de la demande: **02.11.83 Bulletin 83/44**

(45) Mention de la délivrance du brevet: **11.03.92 Bulletin 92/11**

(84) Etats contractants désignés: **AT BE CH DE GB IT LI NL**

(56) Documents cités: **EP-A- 0 063 949**
**FR-A- 2 458 585**

**PROC. NATL. ACAD. SCI. USA, vol. 78, no. 5, mai 1981, pages 2893-2897**

**PROC. NATL. ACAD. SCI. USA**

**GENE, vol. 12, 1980, pages 147-154, Elsevier, North-Holland Biomedical Press, NL**

(73) Titulaire: **INSTITUT PASTEUR**
**28, rue du Docteur Roux**
**F-75715 Paris Cedex 15(FR)**

Titulaire: **CENTRE NATIONAL DE LA RECHER-CHE SCIENTIFIOUE (CNRS)**
**15, Ouai Anatole France**
**F-75007 Paris(FR)**

(72) Inventeur: **Klier, André**
**6, allée Guynemer**
**F-93330 Neuilly/Marne(FR)**
Inventeur: **Rapoport, Georges**
**23 Résidence des Gros Chênes**
**F-91370 Verrieres le Buisson(FR)**
Inventeur: **Dedonder, Raymond**
**3, allée des Pépinières**
**F-92290 Chatenay Malabry(FR)**

PROC. NATL. ACAD. SCI. USA, vol. 79, no. 19, octobre 1982, pages 6065-6069

THE EMBO JOURNAL, vol. 1, no. 7, 1982, pages 791-799, IRL Press Ltd., Oxford, GB.

74 Mandataire: **Gutmann, Ernest et al**
**S.C. Ernest Gutmann - Yves Plasseraud 67,**
**boulevard Haussmann**
**F-75008 Paris(FR)**

## Description

Vecteur apte à transformer des bacillaceae et contenant une Sequence d'ADN codant pour une protéine du cristal ou un polypeptide doué de propriétés insecticides, bacillaceae transformés par de tels ADN, compositions contenant de tels polypeptides.

L invention est relative à des vecteurs aptes à transformer des bacillaceae et contenant une ou plusieurs séquences d'ADN codant pour une protéine de cristal ou un polypeptide doué de propriétés insecticides, à des bacillaceae transformés par de tels vecteurs et à des compositions insecticides contenant lesdites protéines du cristal ou polypeptides.

On désigne sous l'expression "protéines du cristal" des protéines cristallisables ou cristallisées intracellulaires qui sont produites par des cellules du genre *Bacillus thuringiensis* ou analogues, lorsque celles-ci entrent en sporulation. Ces protéines qui,dans *Bacillus thuringiensis* sont formées d'un polypeptide ayant un poids moléculaire de l'ordre de 130.000,peuvent constituer jusqu'à 20 ou 30 % des protéines bactériennes. Les propriétés toxiques de ces protéines du cristal à l'égard d'au moins certaines catégories de larves d'insectes, de papillons ou de moustiques. Les protéines de cristal de certaines de ces souches constituent le principe actif de certaines compositions insecticides qui sont déjà utilisées dans l'agriculture. Il en est en particulier ainsi de la protéine du cristal extraite de *B. thuringiensis* sous-type *kurstaki*, particulièrement efficace à l'égard de lépidoptères. D'autres souches de *B. thuringiensis* sont bien connues. Il en est en particulier ainsi de celles qui sont dénommées *Bacillus thuringiensis* sous-type *tolworthi*, *alesti* et *berliner*. A ce genre de souche se rattachent encore celles connues sous la désignation *Bacillus thuringiensis* sous-type *israelensis*, lesquelles produisent dans leur phase de sporulation une protéine de cristal plus particulièrement efficace contre les diptères, parmi lesquels les moustiques et des mouches noires.

Des études déjà réalisées sur diverses souches de *Bacillus thuringiensis* font apparaître que selon le type de souche le ou les séquences d'ADN codant pour lesdites protéines sont portées soit par le gène, soit par des plasmides, soit encore, comme on a pu le constater dans le cadre de la présente invention,par les deux à la fois, notamment chez *Bacillus thuringiensis* souche *Berliner* 1715.

Dans le cadre de telles études de structures, H.E. SCHNEPF et H.R.WHITELEY ontréussi à isoler un fragment portant une séquence codant pour une protéine de cristal parmi les fragments obtenus par digestion partielle d'un plasmide extrait de *B. thuringiensis* sous-type *kurstaki* HD-1, en présence de l'enzyme de restriction Sau 3A1 (Proc. Natl. Acad. Sci. USA, vol. 78, No. 5, pp. 2893-2897, 1981). Ces auteurs ont décritl'insertion de ce fragment dans le site Bam HI du vecteur de clonage pBR 322 et la transformation par le plasmide recombinant obtenu (pES1)d'une souche d'*Escherichia coli* HB 101. L'incorporation du plasmide recombinant dans *E. coli* a été démontrée par l'expression de l'insérat contenu dans pES1, dans l'une des colonies ( ES 12), sous la forme d'un polypeptide réagissant avec des anticorps préalablement formés contre là protéine du cristal extraite de la même souche initiale de *B. thuringiensis* et, d'autre part leur toxicité à l'égard de chenilles nouvelles nées de certaines espèces de vers infestant les plants de tabac (caterpillars of tobacco hornworm). Le polypeptide formé possédait donc bien des propriétés similaires à celles de la protéine de cristal extraite des souches de *B. thuringiensis* correspondantes. Les quantités produites ont été très faibles, comparées à celles susceptibles d'être produites par *B. thuringiensis*. Selon l'hypothèse formulée par les auteurs, la faible production de polypeptide paraissait devoir être attribuée aux modes de régulation différents de la production de la protéine dans *B. thuringiensis* et *E. coli*.

L'invention a pour but de fournir des moyens permettant de s'affranchir dans des proportions au moins substantielles de ces apparentes difficulté à réaliser l'expression dans un hôte étranger ou hétérologue d'une séquence d'ADN codant pour un polypeptide ayant des propriétés insecticides du type de celles de la protéine de cristal.

L'invention a encore pour but de fournir des séquences d'ADN, notamment des vecteurs, par exemple du type plasmide, susceptibles de transformer des Bacillaceae et de les induire à produire des quantités relativement importantes de polypeptides toxiques présentant des caractéristiques analogues à celles des protéines du cristal, voire même d'autres types de polypeptides toxiques.

A cet égard, l'invention a donc encore pour but la constitution de vecteurs permettant les transformations de bacillaceae, en vue d'induire la production dans ces derniers de polypeptides toxiques, que ceux-ci soient des analogues des différentes protéines de cristal produites par les différentes souches naturelles de *B. thuringiensis* (par exemple *B. thuringiensis berliner*, *kurstaki* ou *israelensis*)ou de polypeptides analogues de protéines naturelles provenant de souches naturelles tout à fait distinctes. L'invention a par conséquent plus particulièrement pour but de fournir des moyens, notamment sous forme vecteurs contenant des séquences d'ADN, et bacillaceae transformés permettant la réalisation de compositions insecticides contenant simultanément un polypeptide s'apparentant à la protéine du cristal et des protéines ou polypeptides insecticides tout à fait distincts, donc de compositions chimiques à spectre d'action insecticide très étendu.

L'invention concerne encore les bacillaceae transformés par des ADN contenant des insérats codant pour la protéine de cristal, plus particulièrement encore de spores de ces micro-organismes hétérologues.

Il est entendu que l'expression "hétérologue", comme elle a été utilisée ci-dessus et comme elle le sera encore ci-après, vise à faire apparaître que le micro-organisme permettant l'expression d'un gène'déterminé est distinct du micro-organisme naturel (homologue) dans lequel le gène en question est normalement exprimé. Cette expression sera également encore utilisée pour désigner une séquence d'ADN (insérat) incorporée, notamment par recombinaison génétique *in vitro,* dans un ADN avec lequel cet insérat ne se trouve pas normalement associé dans la nature.

L'invention a encore pour but de fournir des séquences spécifiques contenant toute l'information génétique des gènes de protéine de cristal, ou du moins une information génétique suffisante pour que le polypeptide résultant de l'expression de ces fragments spécifiques en possède néanmoins les propriétés de toxicité requises en vue de leur utilisation éventuelle postérieure,pour la constitution de compositions insecticides.

L'invention découle de la découverte que des séquences d'ADN contenant un gène ou plus généralement une séquence codant pour une protéine de cristal pouvaient être également exprimées dans des souches de bacillaceae susceptibles d'entrer en sporulation, avec des rendements relativement importants.

L'invention fournit à cet égard des vecteurs de clonage, notamment des plasmides, caractérisés à la fois par leur capacité à transformer des bacillaceae transformables susceptibles d'entrer en sporulation et par le fait qu'ils contiennent eux-mêmes un insérat formé d'une séquence d'ADN codant pour un polypeptide ayant des propriétés toxiques du genre de celles que possèdent les protéines du cristal.

Sous l'expression "bacillaceae transformables", il faut entendre tout bacillaceae hétérologue vis-à-vis de la séquence contenue dans l'insérat susdit, dont est connue ou dont a été acquise la capacité à être transformé par des vecteurs hétérologues. A cette catégorie de bacillaceae transformables appartiennent notamment des *bacillaceae* susceptibles d'entrer en sporulation, tels que *Bacillus subtilis* ou *Clostridium perfringens.*

Il va sans dire que le vecteur devra dans chaque cas présenter les caractéristiques, voire les marqueurs, lui permettant à la fois d'assurer la transformation du bacillaceae transformable susceptible d'être utilisé et de permettre ensuite l'identification ou la reconnaissance des colonies transformées. Au titre des caractéristiques, on peut mentionner les séquences ADN contenues dans ces vecteurs,qui sont susceptibles d'être reconnues par les mécanismes de régulation interne de l'hôte et ses ARN-polymérases, du moins celles produites dans certaines phases du développement de ces micro-organismes.

En ce qui concerne plus particulièrement la séquence d'ADN codant pour le susdit polypeptide et qui se trouve contenue dans l'insérat susdit, elle pourra être caractérisée par sa capacité d'hybridation avec un ADN contenant par nature un gène de la protéine du cristal, qu'il s'agisse d'un fragment chromosomique originaire de la cellule homologue, d'un plasmide également originaire de la cellule homologue, ou encore d'une séquence de sous-fragmentation obtenue à partir des fragments précédents. Au titre des gènes de reconnaissance susceptibles d'être utilisés, on pourra encore mentionner les cADN résultant de la transcription réverse d'un ARN messager, tel qu'il résulte de la transcription du gène codant pour la protéine du cristal en question dans son hôte naturel.

Il résulte d'ailleurs de ce qui précède que la séquence d'ADN contenue dans l'insérat peut elle-même être constituée par un cADN du genre en question.

Conformément à un mode de réalisation préféré de l'invention, les séquences d'ADN susceptibles d'être utilisées pour la constitution des susdits insérats sont issues du gène chromosomique de *Bacillus thuringiensis* souche *berliner* 1715, déposée à la Collection Nationale des Cultures de Micro-organismes (C.N.C.M.) sous le n° I-193,soit encore du gène codant pour la même protéine qui est portée par un plasmide de 42 mégadaltons contenu dans ledit *Bacillus thuringiensis* souche *Berliner 1715.*

On peut encore avoir recours au titre des "gènes de référence" ainsi constitués à tous fragments d'ADN contenant le gène de la protéine de cristal, tel qu'il peut être obtenu à partir du plasmide en question, le cas échéant après son incorporation dans un vecteur. A titre de fragment particulièrement intéressant utilisable à ce titre, on mentionne un fragment de 14 kb délimité par des extrémités Bam HI, tel qu'il peut être obtenu à partir du plasmide de 42 Mdal qui a été mentionné plus haut, voire même encore le fragment plus petit contenu dans le précédent, comportant de l'ordre de 4 kb, délimité par des sites Sst I et Pst I.

Il va de soi qu'on peut utiliser comme gène de référence tout autre gène qui, une fois inséré dans un vecteur approprié, notamment du type plasmide, tel qu'il sera décrit plus loin, sera caractérisé par sa capacité à coder pour la production d'un polypeptide ayant des propriétés toxiques du genre de celles de la protéine de cristal. A cet égard, on pourra avoir recours par exemple à un plasmide du type pES 1, tel qu'il a été mentionné plus haut, et dont l'article de H.E. SCHNEPF et H.R. WHITELEY déjà cité a donné le mode d'obtention.

Naturellement ces "gènes de référence", qui permettent la caractérisation de tous ADN susceptibles d'être mis en oeuvre dans le cadre de l'invention, pour autant qu'ils codent pour une protéine toxique du genre indiqué,sont eux-mêmes susceptibles d'être mis en oeuvre dans les vecteurs selon l'invention agencés de façon à permettre l'expression de ces gènes dans l'hôte présentant les caractéristiques appropriées permettant d'être transformés par le plasmide utilisé.

A titre de vérification supplémentaire de l'appropriation d'une séquence d'ADN à coder pour une protéine toxique,

on pourra encore avoir recours au mode de caractérisation immunologique des polypeptides dont la production aura ainsi été induite, notamment par réaction avec des anticorps préalablement formés contre la protéine de cristal initialement obtenue à partir du micro-organisme dont provenait la séquence d'ADN utilisée, par exemple dans les conditions décrites par H.E. SCHNEPF et H.R. WHITELEY ou encore celles qui seront rappelées plus loin, soit par action directe du polypeptide produit sur des larves ou insectes à l'égard desquels ladite protéine de cristal est réputée particulièrement toxique.

Pour ce qui est du vecteur lui-même, dans lequel est incorporé l'insérat du genre en question, il est constitué par tout type d'ADN, notamment vecteur ou cosmide, qui est capable de transformer un bacillaceae transformable, tels que *Bacillus subtilis*, et de s'y répliquer.

Un vecteur avantageux est constitué par un plasmide connu pour sa capacité à transformer *E. coli*, ce plasmide ayant cependant été modifié par une séquence d'ADN le rendant apte à transformer des bacillaceae aptes à former des spores, ou alternativement il est modifié par une séquence d'ADN permettant l'identification ultérieure de bacillaceae aptes à former des spores (micro-organismes procaryotes), lorsque la transformation étant possible, le problème de l'identification des micro-organismes transformés doit encore être résolu. Dans ce dernier cas, la transformation consiste en l'intégration dans le vecteur, plus particulièrement dans le plasmide, d'un facteur de sélection ou d'un marqueur génétique permettant la caractérisation des colonies transformées.

Le facteur de sélection sera naturellement choisi en fonction de la nature du micro-organisme susceptible d'être transformé par le vecteur choisi. S'agissant de *bacillaceae* tels que *Bacillus subtilis*, on aura avantageusement recours à l'incorporation dans le vecteur, surtout s'il s'agit d'un vecteur connu pour sa capacité de transformer *E. coli* (par exemples du plasmide pBR 322) d'un facteur de sélection susceptible de s'exprimer tant dans des micro-organismes gram⁻ que dans des *bacillaceae*. A titre d'exemple d'un tel facteur, on mentionne le facteur de résistance au chloramphénicol.

Il est à cet égard avantageux d'avoir recours à un plasmide "bi-fonctionnel" capable de se répliquer aussi bien chez *E. coli* que chez *B. subtilis*, tel que le recombinant revertant tétracycline résistant de pBR 322 et du fragment délimité par des extrémités Hind III du plasmide pC 194 provenant de *Staphylococcus Aureus*,

inséré dans le site Hind III de pBR 322. Ce facteur bi-fonctionnel peut se répliquer aussi bien en *E. coli* que dans *B. subtilis*. Il est capable de conférer de la résistance à l'ampicilline, à la tétracycline et au chloramphenicol à *E. coli* et une résistance au chloramphénicol à *Bacillus subtilis*. Une souche de *E. coli* SK 15/92 contenant ce plasmide pHV/33 a été déposée à la C.N.C.M. sous le n° I-191. Les séquences d'ADN qui formeront ultérieurement les susdits insérats peuvent notamment être insérés dans un site Pst I du susdit plasmide, le cas échéant après élongation des extrémités terminales requises des deux brins constitutifs dudit insérat, de part et d'autre de celui-ci par des brins poly(dG) et poly(dC), au moyen d'une déoxynucléotidyle-transférase terminale. Il est avantageux pour réaliser cette insertion d'avoir recours à une séquence d'ADN contenant en outre, en amont du gène de la protéine de cristal (ou de la séquence d'ADN codant pour la protéine de cristal), dans le sens de la transcription d'une séquence contenant le promoteur de la transcription du gène de la protéine de cristal (ou de la séquence hybridable à ce gène), sous le contrôle duquel il est normalement placé dans l'ADN naturel, normalement plasmide, qui normalement le contient. En l'occurrence, ce promoteur sera normalement contenu dans une séquence contenant au plus 150 paires de nucléotides.

Avantageusement, l'insérat est dérivé du fragment 14 kb délimité par des extrémité Bam HI, tel qu'obtenu à partir du plasmide de 42 Mdal contenu dans *Bacillus thuringiensis* souche *berliner* 1715 (C.N.C.M. n° I-193), lequel contient la totalité ou une partie seulement des sites suivants, dans l'ordre qui suit :
Bam HI, Pvu II, Eco RI, Eco RI, Eco RI, Sst I,
Eco RI, Pvu II, Pst I, Sst I, Pst I, Pvu II,
Bgl II, Bam HI.

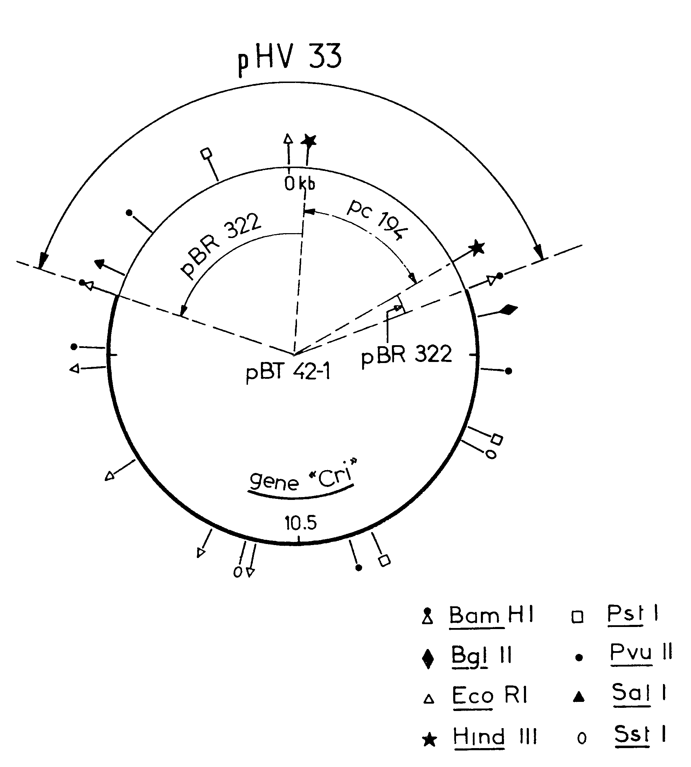

Il a été établi, notamment dans le test immunologique qui sera rappelé plus loin et dans des essais de toxicité vis-à-vis des larves de *Pieris brassicae* que le polypeptide toxique était codé, au moins pour ce qui est de sa plus grande partie, par le fragment contenu dans celui (4 kb) délimité par les sites Sst I et Pst I, comportant donc les sites suivants dans l'ordre qui suit :
Sst I, Eco RI, Pvu II, Pst I(gène"cri" de la fig.1)

Il va de soi que l'invention concerne également des vecteurs contenant des séquences telles que celles qui viennent d'être spécifiées, celles-ci ayant éventuellement subi certaines délétions ou mutations (entraînant par exemple la disparition d'un ou de quelques-uns des sites sus-indiqués), dans la mesure où ces délétions ou mutations n'ont pas néanmoins entraîné la perte des propriétés toxiques des polypeptides susceptibles d'être codés par les séquences de nucléotides ainsi modifiées.

Plus généralement encore l'invention concerne les ADN, notamment plasmides tels qu'ils peuvent être obtenus par recombinaison de plasmides extraits d'un *B. thuringiensis* et d'un autre type de micro-organisme formant des spores, dans la mesure où le recombinant obtenu contient à la fois une séquence codant pour un polypeptide ayant les propriétés toxiques de la protéine du cristal et la capacité de transformer l'autre micro-organisme.

L'invention concerne encore *Bacillaceae* transformés par un ADN contenant une séquence d'ADN codant pour la protéine du cristal ou une protéine toxique analogue, le cas échéant le promoteur qui lui est associé, et le cas échéant encore un promoteur distinct, associé à un facteur susceptible d'être à la fois transcrit et exprimé dans ledit *Bacillaceae*.

Les *Bacillaceae* en question sont avantageusement choisis parmi ceux qui contiennent dans leur propre génome des séquences codant pour une ARN-polymérase et des éléments de régulation permettant la transcription de gènes de sporulation de ces *Bacillaceae*, cette ARN-polymérase et lesdits éléments de régulation étant également capables de reconnaître le gène de la protéine de cristal.

Avantageusement les *Bacillaceae* sont constitués par un *B. subtilis*.

De façon surprenante, il a été constaté que des vecteurs ainsi modifiés étaient capables de transformer efficacement tant des *bacillaceae* que *E. coli* (du moins en ce qui concerne les vecteurs recombinants contenant une partie d'un vecteur tel que pBR 322, dont est connue la capacité de transformation de *E. coli*), d'y être transcrits et exprimés dans des conditions à ce point efficaces qu'il est possible d'observer au microscope optique, de grossissement au moins égal à 500, notamment de l'ordre de 1000, des inclusions de protéines de cristal dans des cultures de *E. coli* ou de *bacillaceae*; notamment *B. subtilis*, lorsque ces derniers sont en cours de sporulation.

L'invention concerne donc également les spores originaires de micro-organismes contenant ledit plasmide et, le cas échéant, lesdites inclusions cristallines.

L'invention concerne aussi les compositions insecticides contenant les susdits bacillaceae conservés à l'état de lyophylisats ou autre forme de conservation, notamment (s'agissant de bacillaceae susceptibles d'entrer en sporulation) à l'état de spores associées le cas échéant à des ingrédients solides permettant la constitution, à l'occasion de leur dilution dans une solution aqueuse, plus particulièrement de l'eau, d'un milieu de culture propice à la germination desdites spores. Lorsque le bacillaceae hôte est constitué par *B. subtilis*, ces ingrédients sont, par exemple, ceux servant à la constitution du milieu de SCHAEFFER et Coll. (selon la référence bibliographique indiquée plus loin). La solution ou suspension ainsi formée peut ensuite être répandue ou dispensée de toutes façons en soi connues sur les végétaux à traiter.

Des caractéristiques supplémentaires de l'invention apparaîtront encore au cours de la description de la production de bacillaceae transformés aptes à produire une protéine toxique les rendant propres à la constitution de compositions insecticides utilisables dans l'agriculture.

1°) Extraction,à partir d'un plasmide de 42 Mdal de *Bacillus thuringiensis* souche *berliner* 1715, d'une séquence Bam HI d'environ 14 kb.

On récupère les ADN de *B. thuringiensis* à partir d'une culture selon la technique de ISCH-HOROWICZ, D. et BURKE J.R. (1981, Nucl. Ac. Res. 9, 2989-2998) ou de BIRNBOÏM, H.C. et DOLY J. (1979, Nucl. Ac. Res. 7, 1513-1523). Les techniques de ces auteurs sont cependant modifiées par addition de sucrose à raison de 20 % en poids au tampon de lyse, pour former des protoplastes. La suspension de cellules est incubée au bain-marie à 37°C pendant 60 minutes et on suit la formation de protoplastes en microscopie à contraste de phase. Les plasmides ont été purifiés dans un gradient isopycnique de chlorure de césium. On procède à une électrophorèse des fractions ADN obtenues sur des plaques horizontales à base de gel d'agarose de 0,7 ou 0,5 %. Le tampon d'électrophorèse utilisé est constitué par du Tris 89 mM, de l'acide borique 89 mM, Na$_2$ EDTA 2,5 mM, pH 8,3, pendant 16 heures sous 3 volts/cm. On sépare les plasmides par électrophorèse en gel apres visualisation des bandes au moyen du bromure d'éthidium 1 microgramme/ml (bandes photographiées sous irradiation ultra-violette). On traite les bandes correspondant au plasmide de 42 Mdal avec l'enzyme de restriction Bam HI et on sépare les fragments ayant une taille d'environ 14 kb.

2°) Construction du vecteur pBT 42-1 résultant de l'insertion de la séquence de 14 kb extraite du susdit plasmide, lui-même obtenu de *B. thuringiensis* dans le site Bam HI de pHV 33.

La figure unique jointe est une carte de restriction du plasmide final obtenu. Elle fait apparaître, dans le recombinant obtenu, la partie originaire de pHV 33 (arc de cercle moins épais) et le susdit fragment de 14 kb (arc de cercle plus épais).

Les arcs de cercle fléchés font également apparaître les positions relatives dans pHV 33 des séquences d'ADN respectivement originaires de pBR 322 (arc de cercle fin en continu) et de pC 194 (arc en trait mixte). La partie correspondant à la séquence de pC 194 contient le gène de résistance au chloramphénicol et le promoteur qui lui est associé.

La préparation de pBT 42-1 a été réalisée comme suit :

2 microgrammes du digestat de Bam HI des plasmides extraits de *B. thuringiensis* et 1 microgramme de pHV 33 préalablement linéarisé ont été incubés pendant 1 heure à 20°C au sein d'un tampon approprié pendant 16 heures à 8°C, en présence de 0,25 unité de DNA-ligase de T$_4$ (de BOEHRINGER), dans un volume de 25 microlitres. Des portions aliquotes (2 microlitres) du mélange de ligation ont été utilisées pour transformer la souche de *E. coli* K12 HB 101 (C.N.C.M. n° I-190).

D'autres aliquotes ont été utilisées pour transformer *B. subtilis* souche QB 666 (C.N.C.M. I-192), par exemple selon une technique connue décrite par AGNOS TOPOVLOS C. et SPIZIZEN J. (1961, J. Bacteriol. 81, 741-746). Les cellules contenant des plasmides résistant aux antibiotiques ont été sélectionnées sur des plaques d'agar L additionné avec soit de l'ampicilline (100 microgrammes/ml), soit de la tétracycline (15 microgrammes/ml) ou du chloramphénicol (5 microgrammes/ml).

Le taux de transformation de la souche de *B. subtilis* est assez élevé. On repère les souches recombinantes qui font apparaître l'intégration d'une séquence d'ADN. On met en culture les souches transformées de *B. subtilis* dans des conditions conduisant à sa sporulation, par exemple dans un milieu tel que décrit par SCHAEFFER P., IONESCO A., RITER A., DALASSA G. dans l'article intitulé "Mécanismes de régulation des activités cellulaires chez les micro-organismes", Editions du C.N.R.S., 553-563, pour favoriser la synthèse d'un polypeptide ayant des propriétés toxiques du type de celles de la protéine du cristal. Les cellules de *B. subtilis* effectivement transformées par un plasmide contenant le gène codant pour la protéine du cristal se sont révélées effectivement contenir des inclusions protéiques ayant des tailles de 0,05 à 0,2 micron (mesurées au microscope électronique sur des extraits obtenus par sonication des cellules).

On a de la même manière constaté que les cellules de *E. coli* effectivement transformées par un plasmide contenant le gène de la protéine du cristal avaient produit une inclusion visible sous microscope optique (grossissement 1000). Les cristaux présentaient des tailles de l'ordre de 0,05 à 0,5 micron (mesurées dans les conditions sus-indiquées).

Les quantités de protéine produites sous forme d'inclusion atteignent jusqu'à 10 % de la quantité formée chez *B. thuringiensis,* et ce chez *E.coli* comme chez *B. subtilis.*

3°) Détection des polypeptides exprimés.

On provoque la rupture des cellules et on recueille les protéines et polypeptides formés. On les sépare dans un gel à gradient exponentiel de 7,5 à 15 % selon la technique de O'FARRELL, P.H. (1975, J. Biol. Chem., 250, 4007-4001). Les polypeptides sont transférés sur des filtres de nitrocellulose selon la méthode de BOWEN B. et al (1980, Nucl. Ac. Res., 8, 1-20) La réaction avec des anticorps préalablement formés à l'égard de la protéine de cristal a été effectuée par une méthode du type de celle décrite par H.E.SCHNEPF et al, indiquée plus haut.

Les polypeptides formés se sont également révélés particulièrement toxiques à l'égard des larves de *Pieris brassicae.* Ceci a été mis en évidence par mise en contact de ces chenilles nouvellement nées avec des extraits de *E. coli* et de *B. subtilis,* respectivement transformés. Le milieu obtenu a été étalé sur des petites feuilles de chou qui ont été données aux larves. Une proportion considérable de ces larves avaient péri 18 heures après l'alimentation à la température ambiante.

D'une façon générale, on peut encore faire les remarques suivantes à l'égard des résultats que l'invention a permis d'obtenir.

Il est significatif que les insérats contenus dans les plasmides,eux-mêmes contenus dans les cellules ayant produit des cristaux, décelables sous microscope optique, de polypeptides toxiques, étaient hybridables à des plasmides contenant des gènes codant pour la protéine du cristal, tels qu'obtenus à partir d'autres souches de *B. thuringiensis,* notamment des souches *berliner* 22105, *kurstaki* HD1 et *subtoxicus*. Il est d'ailleurs significatif qu'il a été possible d'extraire un fragment d'environ 6 kb contenant le gène codant pour la protéine de cristal et respectivement délimité par des extrémités Sst-1, à partir de ces autres souches. Les poids moléculaires des polypeptides synthétisés par les susdites cellules se sont révélés être du même ordre de grandeur que celles de la protéine de cristal.

Les cellules de *B. subtilis* se sont avérées ne synthétiser les quantités substantielles susindiquées de polypeptides toxiques que lorsqu'elles se trouvaient en phase de sporulation, d'où l'analogie vraisemblable ainsi mise en évidence entre les ARN-polymérases et les éléments de régulation permettant la transcription des gènes de sporulation chez *B. subtilis* et *B. thuringiensis*.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes.

**Revendications**

1. Vecteur apte à transformer des bacillaceae transformables hétérologues par rapport à B. thuringiensis, caractérisé par le fait qu'il contient lui-même un insérat qui comporte une séquence d'ADN codant pour une protéine du cristal de B. thuringiensis ou pour une partie de cette protéine ayant des propriétés insecticides d'une protéine de cristal, et que cette séquence d'ADN est, au sein de ce vecteur, associée à des éléments de régulation autorisant sa transcription et son expression, sous la forme d'inclusions cristallines de protéine du cristal, dans lesdits bacillaceae.

2. Vecteur selon la revendication 1, caractérisé par sa capacité à se répliquer dans Bacillus subtilis.

3. Vecteur selon la revendication 1 ou la revendication 2, caractérisé en ce que le susdit insérat contient aussi le promoteur qui est normalement associé à ladite séquence d'ADN dans l'ADN homologue, notamment plasmide, dont elle est issue.

4. Vecteur selon l'une quelconque des revendications 1 à 3, caractérisé en ce que le susdit insérat est originaire d'un plasmide porteur du gène de la protéine du cristal dans B. thuringiensis.

5. Vecteur selon l'une quelconque des revendications 1 à 4, caractérisé par sa capacité à également transformer E. coli.

6. Vecteur selon la revendication 1 ou la revendication 3, caractérisé par le fait qu'il contient un facteur de sélection, tel que le facteur de résistance au chloramphénicol, susceptible de s'exprimer tant dans des micro-organismes gram⁻ que dans des bacillaceae, tels que B. subtilis.

7. Plasmide selon l'une quelonque des revendications 1 à 6, caractérisé en ce que l'insérat susdit est constitué par une séquence d'ADN constitué par une séquence d'ADN provenant d'un gène de protéine de cristal, extrait de B. thuringiensis contenant la totalité ou une partie seulement des sites dans l'ordre qui suit :
Bam HI, Pvu II, Eco RI, Eco RI, Eco RI, Sst I, Eco RI, Pvu II, Pst I, Sst I, Pst I, Pvu II, Bgl II, Bam HI.

8. Plasmide selon la revendication 7, caractérisé en ce que l'insérat susdit contient au plus 14 kb.

9. Plasmide selon la revendication 6, caractérisé en ce que l'insérat comporte des sites dans l'ordre suivant :
Sst I, Eco RI, Pvu II, Pst I.

10. Plasmide selon la revendication 9, caractérisé en ce que l'insérat a une taille de l'ordre de 4 kb.

11. Plasmide selon l'une quelconque des revendications 7 à 10, caractérisé en ce qu il contient le gène de la protéine de cristal et, en amont de ce gène dans le sens de la transcription, une séquence contenant au plus 150 paires de nucléotides correspondant à la séquence de nucléotides qui, dans le plasmide naturel dont le gène de la protéine du cristal est issu, contient le promoteur de la transcription du gène de protéine de cristal, sous le contrôle duquel il est normalement placé.

12. Bacillaceae, aptes à former de spores, hétérologue vis-à-vis de B. thuringiensis, ce bacillaceae étant transformé par une séquence ADN codant pour un polypeptide possédant des propriétés insecticides d'une protéine du cristal de B. thuringiensis et, le cas échéant, le promoteur qui lui est associé, et ce sous le contrôle d'éléments de régulation autorisant la transcription et l'expression de cet ADN codant, sous la forme d'inclusions cristallines de protéine du cristal, lorsque ces bacillaceae sont mis en culture.

13. Bacillaceae selon la revendication 12, caractérisé en ce qu'il contient un ADN comprenant également un promoteur distinct, associé à un facteur de sélection susceptible d'être à la rois transcrit et exprimé dans ledit bacillaceae.

**14.** Bacillaceae selon la revendication 13, caractérisé en ce que le facteur de sélection est un facteur de résistance à un antibiotique, tel que le chloramphénicol.

**15.** Bacillaceae selon la revendication 13 ou la revendication 14, caractérisé en ce qu'il contient dans son génome des séquences codant pour une ARN-polymérase et des éléments de régulation permettant la transcription de gènes de sporulation de ce micro-organisme, cette ARN-polymérase et lesdits éléments de régulation étant également capables de reconnaître le gène de protéine de cristal.

**16.** Bacillaceae selon l'une quelconque des revendications 12 à 15, caractérisé en ce qu'il est constitué par B. subtilis.

**17.** Bacillaceae selon les revendications 12 à 16 contenant des inclusions de protéine de cristal, visibles au microscope optique de grossissement au moins égal à 500, notamment de l'ordre de 1000.

**18.** Les spores originaires des bacillaceae contenant ledit plasmide, selon l'une quelconque des revendications 12 à 17.

**19.** Composition insecticide contenant un lyophilisat ou autre forme de conservation d'un bacillaceae conforme à l'une quelconque des revendications 12 à 17.

**20.** Composition contenant les spores selon la revendication 18, le cas échéant associées à des ingrédients solides permettant la constitution, à l'occasion de leur dilution au sein de l'eau, d'un milieu de culture propice à la germination desdites spores.

**Claims**

**1.** Vector able to transform transformable bacillaceae heterologous with respect to B.thuringiensis, characterized by the fact that it contains itself an insert which comprises a DNA sequence coding for a crystal protein of B. thuringiensis or for a part of said protein having insecticidal properties of a crystal protein, and in that said DNA sequence is, within said vector, associated with regulation elements authorizing its transcription and its expression, in the form of crystalline inclusions of crystal protein in said bacillaceae.

**2.** Vector according to claim 1, characterized by its capability to replicate in Bacillus subtilis.

**3.** Vector according to claim 1 or to claim 2, characterized in that said insert contains also the promoter which is normally associated with said DNA sequence in the homologous DNA, particularly plasmid, from which it originated.

**4.** Vector according to anyone of claims 1 to 3, characterized in that said insert originates from a plasmid which carries the gene of the crystal protein on B.thuringiensis.

**5.** Vector according to anyone of claims 1 to 4, characterized by its capability of also transforming E.coli.

**6.** Vector according to claim 1 or to claim 3, characterized by the fact that it contains a selection factor such as the chloramphenicol resistance factor, liable of being expressed in gram microorganisms as well as in bacillaceae, such as B.subtilis.

**7.** Plasmid according to anyone of claims 1 to 6, characterized in that said insert is constituted by a DNA sequence originating from the gene of a crystal protein, extracted from B.thuringiensis containing all or only part of the sites in the order which follows:
Bam HI, Pvu II, Eco RI, Eco RI, Eco RI, Sst I, Eco RI, Pvu II, Pst I, Sst I, Pst I, Pvu II, Bgl II, Bam HI.

**8.** Plasmid according to claim 7, characterized in that said insert contains at the most 14 kb.

**9.** Plasmid according to claim 6, characterized in that the insert contains sites in the following order:
Sst I, Eco RI, Pvu II, Pst I.

**10.** Plasmid according to claim 9, characterized in that the insert has a size of about 4 kb;

**11.** Plasmid according to anyone of claims 7 to 10, characterized in that it contains the gene of the crystal protein and, upstream from that gene in the direction of transcription, a sequence containing at the most 150 pairs of nucleotides corresponding to the sequence of nucleotides which, in the natural plasmid from which the gene of the crystal protein originates, contains the promoter of the transcription of the gene of the protein crystal, under the control of which it is normally located.

**12.** Bacillaceae, capable of forming spores, heterologous with respect to B.thuringiensis, said bacillaceae being transformed by a DNA sequence coding for a polypeptide possessing insecticidal properties of a crystal protein of B.thuringiensis and, if appropriate, the promoter associated therewith, and that under the control of regulation elements authorizing the transcription and expression of said coding DNA, in the form of crystalline inclusions of crystal protein, when these bacillaceae are brought into culture.

13. Bacillaceae according to claim 2, characterized in that it contains a DNA which equally comprises a distinct promoter, associated with a selection factor capable of being both transcribed and expressed in said bacillaceae.

14. Bacillaceae according to claim 13, characterized in that the selection factor is a factor of resistance to an antibiotic, such as chloramphenicol.

15. Bacillaceae according to claim 13 or 14, characterized in that it contains in its genome sequences coding for a RNA-polymerase and regulation elements authorizing the transcription of the sporulation genes of said microorganism, said RNA-polymerase and said regulation elements being also capable of recognizing the gene of the crystal protein.

16. Bacillaceae according to anyone of claims 12 to 15, characterized in that it is constituted by B.subtilis.

17. Bacillaceae according to claims 12 to 16, containing inclusions of crystal protein, which can be viewed under an optical microscope having a magnifying power equal at least to 500, particularly of the order of 1000.

18. The spores which originate from bacillaceae containing said plasmid according to anyone of claims 12 to 17.

19. Insecticidal composition containing a lyophilisate or another form of conservation of a bacillaceae according to anyone of claims 12 to 17.

20. Composition containing the spores according to claim 18, associated with an appropriate solid ingredient permitting the forming, when diluted in water, of a culture medium favoring the germination of said spores.

**Patentansprüche**

1. Vektor geeignet zum Transformieren von in Bezug auf B. thuringiensis heterologen transformierbaren Bacillaceae, dadurch gekennzeichnet, daß er selbst eine Insertion enthält, die eine DNA-Sequenz umfaßt, die für ein Kristallprotein von B. thuringiensis kodiert oder für einen Teil dieses Proteins, der die insektiziden Eigenschaften eines Kristallproteins aufweist, und daß diese DNA-Sequenz in diesem Vektor mit Regulationselementen verbunden ist, die ihre Transkription und ihre Expression in Form von kristallinen Einschlüssen von Kristallprotein in den genannten Bacillaceae erlauben.

2. Vektor nach Anspruch 1, gekennzeichnet durch seine Fähigkeit, sich in Bacillus subtilis zu replizieren.

3. Vektor nach Anspruch 1 oder 2, dadurch gekennzeichnet, daß die genannte Insertion auch den Promotor enthält, der normalerweise mit der genannten DNA-Sequenz in der homologen, insbesondere plasmiden DNA, aus der sie abstammt, verbunden ist.

4. Vektor nach einem der Ansprüche 1 bis 3, dadurch gekennzeichnet, daß die genannte Insertion von einem Trägerplasmid des Gens des Kristallproteins in B. thuringiensis stammt.

5. Vektor nach einem der Ansprüche 1 bis 4, gekennzeichnet durch seine Fähigkeit, gleichermaßen E. coli zu transformieren.

6. Vektor nach Anspruch 1 oder 3, dadurch gekennzeichnet, daß er einen Selektionsfaktor enthält, wie den Resistenzfaktor für Chloramphenicol, der fähig ist, sich sowohl in gram-negativen Mikroorganismen als auch in Bacillaceae wie B. subtilis zu exprimieren.

7. Plasmid nach einem der Ansprüche 1 bis 6, dadurch gekennzeichnet, daß die genannte Insertion aus einer DNA-Sequenz gebildet ist, die aus einer DNA-Sequenz gebildet ist, die von einem Gen des Kristallproteins herstammt, die von B. thuringiensis extrahiert ist und die gesamten oder nur einen Teil der Stücke in der folgenden Ordnung enthält:

BAM HI, Pvu II, Eco RI, Eco RI, Eco RI, Sst I, Eco RI, Pvu II, Pst I, Sst I, Pst I, Pvu II, Bgl II, Bam HI.

8. Plasmid nach Anspruch 7, dadurch gekennzeichnet, daß die genannte Insertion höchstens 14 kb enthält.

9. Plasmid nach Anspruch 6, dadurch gekennzeichnet, daß die Insertion Stücke in der folgenden Ordnung umfaßt:

Sst I, Eco RI, Pvu II, Pst I.

10. Plasmid nach Anspruch 9, dadurch gekennzeichnet, daß die Insertion eine Größe in der Größenordnung von 4 kb aufweist.

11. Plasmid nach einem der Ansprüche 7 bis 10, dadurch gekennzeichnet, daß es das Gen des Kristallproteins enthält und oberhalb dieses Gens in Richtung der Transkription eine Sequenz, die höchstens 150 Nukleotidpaare entsprechend der Nukleotidsequenz enthält, die im natürlichen Plasmid, aus dem das Gen des Kristallproteins abstammt, den Promotor der Transkription des Gens des Kristallproteins enthält, unter dessen Kontrolle es normalerweise gestellt ist.

12. Bacillaceae geeignet zur Bildung von Sporen, heterolog gegenüber B. thuringiensis, wobei diese Bacillaceae durch eine DNA-Sequenz transformiert werden, die für ein Polypeptid, das insektizide Eigenschaften eines Kristallproteins von B. thuringiensis besitzt und gegebenenfalls den mit ihm verbundenen Promotor kodiert, und dies unter der Kontrolle von Regulationselementen, die die Transkription und Expression dieser kodierenden DNA in Form von kristallinen Einschlüssen von Kristallprotein erlauben, wenn diese Bacillaceae in Kultur gebracht werden.

13. Bacillaceae nach Anspruch 12, dadurch gekennzeichnet, daß sie eine DNA enthält, die gleichermaßen einen bestimmten Promotor umfaßt, verbunden mit einem Selektionsfaktor, der geeignet ist, im genannten Bacillaceae gleichzeitig transkribiert und exprimiert zu werden.

14. Bacillaceae nach Anspruch 13, dadurch gekennzeichnet, daß der Selektionsfaktor ein Resistenzfaktor für ein Antibiotikum wie das Chloramphenicol ist.

15. Bacillaceae nach Anspruch 13 oder 14, dadurch gekennzeichnet, daß es in seinem Genom Sequenzen enthält, die für eine RNA-Polymerase kodieren und Regulationselemente, die die Transkription von Genen zur Sporenbildung dieses Mikroorganismus erlauben, wobei diese RNA-Polymerase und die genannten Regulationselemente gleichermaßen fähig sind, das Gen des Kristallproteins zu erkennen.

16. Bacillaceae nach einem der Ansprüche 12 bis 15, dadurch gekennzeichnet, daß es aus B. subtilis gebildet ist.

17. Bacillaceae nach einem der Ansprüche 12 bis 16, die Einschlüsse von Kristallprotein enthalten, die im optischen Mikroskop bei einer Vergrößerung von mindestens gleich 500, insbesondere in der Größenordnung von 1000, sichtbar sind.

18. Sporen, die von Bacillaceae stammen, die das genannte Plasmid enthalten, nach einem der Ansprüche 12 bis 17.

19. Insektizide Zusammensetzung, die ein Lyophilisat oder eine andere Form von Konservierung eines Bacillaceae gemäß einem der Ansprüche 12 bis 17 enthält.

20. Zusammensetzung, die die Sporen nach Anspruch 18 enthält, gegebenenfalls verbunden mit festen Bestandteilen, die durch ihre Verdünnung in Wasser die Bildung eines Kulturmilieus erlaubt, das für die Germination der genannten Sporen günstig ist.

pHV 33

Bam HI    Pst I

Bgl II    Pvu II

Eco RI    Sal I

Hind III    Sst I